# EUROPEAN PATENT APPLICATION

(11) **EP 0 741 134 A1**
(43) Date of publication of application: **06.11.1996**
(21) Application number: 96105414.5
(22) Date of filing: 04.04.1996
(51) Int. Cl.: C07D 303/16, C08F 255/00

(54) **Unsaturated glycidyl esters and their use as polyolefin modifiers**

(30) Priority: 05.05.1995 IT MI950898
(71) Applicant: ENICHEM S.p.A., I-20124 Milano (IT)
(72) Inventor: Pastorino, Maria Antonella, Genova (IT); Schimperna, Giuliana, Milano (IT); Caldararo, Maria, Trecate (NO) (IT); Gennaro, Antonio, Cameri (IT)
(74) Representative: Gennari, Marco

(57) **Abstract**

The present invention relates to glycidyl esters containing an unsaturation, the process for their preparation and their use for the preparation of modified polyethylenes.
Glycidyl esters have the following general formula: 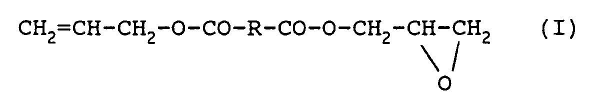 wherein R can be an aromatic ring diversely subsituted as phenyl or naphthyl or a linear or branched aliphatic chain formed by 4 to 20 carbon atoms, or norbornan or furan excluding 1-allyl-2-oxyran-2-ylmethyl-terephthalate.
Polyolefins modified by radicalic functionalization with these esters have improved adhesion properties to metals.

## Description

The present invention relates to glycidyl esters containing an unsaturation, the process for their preparation and their use for the preparation of modified polyethylenes.

The invention also relates to polyolefins modified by radicalic functionalization with glycidyl esters and the functionalization process.

Polyolefins are non-polar products which have a limited adhesion and affinity with respect to glass, metals and polar polymers.

The preparation of composite or laminated materials starting from polyolefins and from the above materials is therefore extremely difficult.

For this reason, in the last few years intensive research has been carried out to obtain polyolefins with improved surface properties.

Numerous examples of functionalization reactions of polyolefins with diesters of unsaturated bi-carboxylic acids, unsaturated epoxides and unsaturated glycidyl esters are provided in literature.

U.S. patent 4.147.740 of Swiger et al. for example, describes polyethylene modified with derivatives of maleic, itaconic and citraconic acid and the process for their preparation.

The article by Achintya et al. published in 1991 (Angewandte Makromoleculare Chemie, 191, 15-30) describes, in turn, the grafting of dibutylmaleate on polyethylene (PE) and on the terpolymer based on ethylene, propylene and ethylidene norbornene (EPDM).

The reactivity in the grafting reaction of these diesters however is low, and this corresponds to a low functionalization degree and to a limited improvement in the surface properties.

The grafting process of glycidyl esters, in particular glycidylacrylate and glycidylmethacrylate, on general polymers is described in U.S. patent 5.066.726.

It is also known that adhesives based on epoxyalkyl esters are materials of interest owing to their good adhesion characteristics with respect to glass, marble and metals (C.A.A.Rayner in "Adhesion and adhesives", vol.I, chap.4, page 277, Ed. Houwink and Salomon, Eslevier, 1965.

We have now found that the use of particular glycidyl esters for the modification reaction of polyolefins enables the production of materials which, with the same reacted grafting moles, have much better adhesion properties to metals than those of materials modified with glycidylmethacrylate.

In accordance with this a first object of the present invention relates to a glycidyl ester containing an unsaturation, having the following general formula: 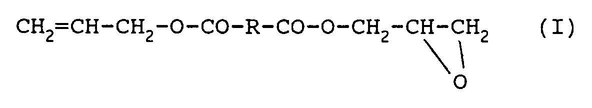 wherein R can be an aromatic ring diversely substituted as phenyl or naphthyl or a linear or branched aliphatic chain formed by 4 to 20 carbon atoms, or norbornan or furan.

A second object of the present invention relates to a method for the preparation of the glycidyl esters having formula (I) which consists in the monoepoxidation of the corresponding diallylesters, by reaction in phase transfer with hydrogen peroxide at 35% (m/v) and in the presence of catalysts based on tungsten.

A third object of the invention relates to a modified polyolefin which can be obtained by the radicalic grafting reaction in the molten state of a polyolefin having a density of at least 0.85, preferably between 0.86 and 0.97, with 0.1-10% by weight with respect to the polyolefin of a glycidyl ester having formula (I), in the presence of 0.01-0.50% by weight of an initiator, consisting of an organic peroxide.

Finally, a fourth object of the invention relates to a process for the preparation of this modified polyolefin characterized in that 100 parts by weight of polyolefin are reacted, in the molten state, with 0.1-10 parts of glycidyl ester having formula (I), for a time ranging from a few seconds to 20 minutes, at a temperature of between 160° and 200°C and in the presence of 0.01 - 0.5 parts of an organic peroxide.

The unsaturated glycidyl esters having formula (I) have a high boiling point, are thermally stable and do not homopolymerize under the conditions used in the functionalization reaction.

The modified polyolefins of the present invention can be used as such as adhesive resins or they can form part of formulations which contain non-modified polyolefins and inorganic phases such as glass fibres, mica, kaolin, etc. They can be used as linings or in the construction of laminates as a binder with respect to metals.

In addition, as the epoxidic functionalities are capable of reacting with carboxyl groups, they can be applied in the development of polymeric alloys with macromolecules having carboxyl chain-ends, for example polyesters or polyamides.

The glycidyl esters of the present invention were prepared by the monoepoxidation of the corresponding diallylesters, by reaction in phase transfer with hydrogen peroxide at 35% (m/v) and in the presence of catalysts based on tungsten, according to the method described by Venturello et al., in Journal of Organic Chemistry (vol.53, page 1553, (1988)). The synthesis procedure of these esters is described in example 1.

The modification reaction of the polyolefins takes place at a temperature of between 160° and 200°C, in the presence of an organic peroxide and in the molten state. At the end of the reaction, antioxidants, stabilisers, nucleating agents, organic and inorganic dyes or other additives, can be added if necessary.

In particular, the modification reaction can be indifferently carried out in a reactor suitable for the purpose or in a static mixer or in an extruder at temperatures ranging from 160° to 200°C, with a preferred range of 170°- 190°C.

There are no elements which can prejudicially exclude the mixing of the grafting substrate and initiator before feeding into the mixer or extruder. If the grafting agent has a melting point lower than room temperature it is preferable however to feed it mixed with the initiator, directly into the molten mass of the substrate and at the reaction temperature. If, on the other hand, the melting point of the grafting agent is higher than room temperature it is preferable to disperse it with a low-boiling grafting solvent and initiator on a fraction of polyolefin, and then feed the mixture obtained by evaporation of the solvent directly into the molten mass of the remaining substrate.

In the reaction mixture the different components are present in the following proportions: grafting agent from 0.1 to 10 parts by weight, initiator from 0.01 to 0.5 parts by weight out of 100 parts of substrate. The reaction is carried out for a time ranging from a few seconds to twenty minutes. Higher times are not excluded provided they do not cause degradation of the substrate.

In the embodiment of the invention the contact times between the ester and polyolefin range from 1 to 10 minutes.

The following glycidyl esters are preferably used:
1-allyl- 4-oxyran-2-ylmethylterephthalate (VEEP01), 1-allyl- 8-oxyran-2-ylmethyloctandioate (VEEP02), 1-allyl-3-oxyran-2-ylmethylterephthalate (VEEP03), 1-allyl-2-oxyran-2-ylmethylterephthalate (VEEP04), allylester, oxyran-2-ylmethylester of bicyclo[2.2.1] heptane2,3-dicarboxylic acid (VEEP05), allylester, oxyran-2-ylmethylester of 2,5-furandicarboxylic acid (VEEP06).

In the context of the invention the term "polyolefin" comprises both homopolymers of ethylene and copolymers of ethylene with propylene, butene and other unsaturated aliphatic hydrocarbons.

The preferred copolymers of ethylene contain up to 50% of higher members of the group of olefins such as propylene, 1-butene, 4-methyl-1-pentene, 1-hexene, 1-octene.

In addition, the copolymers can contain up to 5% of diolefins or triolefins which are commonly used in ethylene/propylene terpolymers such as ethylidenenorbornene, methylenenorbornene, 1,4-hexadiene vinylnorbornene.

The term polyolefin therefore comprises both polymers which are known with the abbreviations HDPE, LLDPE, VLDPE and ULDPE and elastomeric ethylene/propylene copolymers which are known with the abbreviations EPM and EPDM.

Preferred polyolefins are known on the market under the trade-name of ERACLENE^{R}, FLEXIRENE^{R} and RIBLENE^{R} (HDPE, LLDPE and LDPE) and DUTRAL^{R} (EPM and EPDM) and are produced by ENICHEM and ENICHEM ELASTOMERI respectively.

Any inorganic peroxide can be used as initiator, which has an average life time of at least 50 seconds at a temperature within the range specified by the invention.

Usually peroxides represented by the general formula (II) are used: 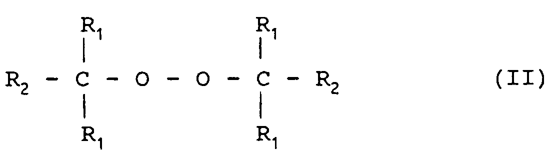 Wherein R₁ is a phenyl radical or alkyl radical containing from 1 to 10 carbon atoms, also unsaturated owing to the presence of double or triple C-C bonds, possibly substituted with alkyl or peroxyalkyl groups containing from 1 to 10 carbon atoms and R₂ is an alkyl radical containing from 1 to 10 carbon atoms.

Examples of initiators which can be used are dicumylperoxide, 2,5-dimethyl-2,5-di(t-butylperoxy) hexane, 2,5-diemthyl-2,5-di(t-butyl-peroxy)-3-hexyne, di-t-butylperoxide.

The initiators used are AKZO products, known under the trade-name of Perkadox and Trigonox respectively.

The following examples provide a further illustration of the invention but do not limit its scope in any way.

### EXAMPLE 1

### Synthesis of glycidyl esters

31.0 g of diallylester, 27.0 ml of 1,2-dichloroethane, 9.0 ml of water and 2.25 g of catalyst based on tungsten are charged into a reactor equipped with a mechanical stirrer, thermometer and drip-funnel. 13.5 ml of hydrogen peroxide (35% m/v) are added drop-wise to the reaction mixture at 50°C. After the dripping, the temperature is brought to 70°C and is maintained as such for about 4 hours. After this period all the hydrogen peroxide has been consumed. The reaction mass is washed with water, the organic phase is anhydrified on sodium sulphate, the solvent is removed by distillation under vacuum.

The product thus obtained is purified by silica gel chromatography (eluant = hexane/ethylacetate = 9/1). 10 g of the starting diester and 17 g of the desired product are thus obtained (yield of the convertited product equal to 72%).

### EXAMPLE 2

25.7 g of LLDPE (Flexirene FG20, produced by EniChem) were fed in an inert atmosphere into a Brabender type static mixer, preheated to a temperature of 180°C. After waiting for the time necessary to melt the product (4 minutes) a further 6 g of LLDPE were charged, on which a mixture consisting of 0.04 g of dicumylperoxide and 3.17 g of 1-allyl,4-oxyran-2-ylmethyl-terephthalate (VEEP01) had been previously dispersed. After about 5 minutes the material was removed from the mixer and cooled. The modification which had taken place was verified on film compression moulded at 180°C and extracted with acetone to remove the non-reacted product by infra-red analysis.

The quantity of grafted ester was then measured on the sample by titration of the epoxy groups with perchloric acid and the peel strength value was determined at 180°C on aluminium. These values are shown in Table 1 together with the characterization data relating to the products of the modification reactions described in the following examples.

### EXAMPLE 3

Example 2 was repeated to evaluate the repeatability of the test.

### EXAMPLE 4

31.7 g of LLDPE were fed into a Brabender mixer, in an inert atmosphere, after heating the apparatus to 180°C. After the 4 minutes necessary for melting the material, a mixture containing 0.15 g of di-t-butyl-peroxide (TBP) and 3 g of 1-allyl-8-oxyran-2-ylmethyl-octandioate (VEEP02) was injected into the molten mass.

After about 5 minutes the material was removed from the mixer and cooled. The subsequent treatments on the sample thus obtained were carried out using the procedure described in example 2.

### EXAMPLE 5

The same procedure was used as in example 4, but the modification was carried out using 31.8 g of LLDPE to which a mixture containing 3 g of VEEP02 and 0.04 g of dicumylperoxide had been added. A material was obtained containing 4.2 g (0.014 moles) of VEEP02 bound onto 100 g of product, with a measured peel strength value of 41 N/cm.

### EXAMPLE 6 (comparative)

The modification reaction described in example 5 was repeated substituting VEEP02 with glycidylmethacrylate. A material was obtained containing 2.0 g (0.014 moles) of glycidylmethacrylate bound onto 100 g of product with a measured peel strength value of 25 N/cm.

### EXAMPLE 7

The modification was carried out according to the procedure described in example 4 with a grafting mixture consisting of 2.7 g of 1-allyl-3-oxyran-2-ylmethylterephthalate (VEEP03) and 0.03 g of dicumylperoxide on 31.8 g of LLDPE.

### EXAMPLE 8

The procedure of example 4 was carried out using a mixture consisting of 2.7 g of 1-allyl-2-oxyran-2-ylmethyl-terephthalate (VEEP04) and 0.07 g of dicumylperoxide.

### EXAMPLE 9

The modification reaction was carried out according to the procedure described in example 4 on 31.7 g of LLDPE FG20 using a grafting mixture consisting of 2.9 g of allyl ester, allylester,oxyran-2-ylmethylester of 2,5-furandicarboxylic acid and 0.04 g of dicumylperoxide.

**TABLE 1**

| | Density^{a} (g/ml) | Peel strength^{b} (N/cm) | Bound ester^{c} (g/100) | Grafting yield (%) |
|---|---|---|---|---|
| LLDPE FG 20 | 0.922 | 0 | - | - |
| Example 2 | 0.929 | 34 | 3.2 | 32 |
| Example 3 | 0.928 | 38 | 3.0 | 30 |
| Example 4 | 0.929 | 30 | 4.1 | 43 |
| Example 5 | 0.926 | 41 | 4.2 | 44 |
| Example 6 | 0.926 | 25 | 2.0 | 21 |
| Example 7 | 0.927 | 38 | 3.1 | 36 |
| Example 8 | 0.928 | 31 | 3.3 | 39 |
| Example 9 | 0.925 | 31 | 1.3 | 14 |

| | | | | |
|---|---|---|---|---|
| ^{a} = Measured according to ASTM 1505 on melted samples, then crystallized for 1 hr at 100°C | | | | |
| ^{b} = Measurement carried out on samples compression moulded between two Al sheets having a thickness of 0.1 mm, with a separation rate of the clamps of 100 mm/min. | | | | |
| ^{c} = Titration with perchloric acid, in a mixed xylene/acetic acid solvent at 110°C | | | | |

## Claims

1. Glycidyl esters having the following general formula (I) 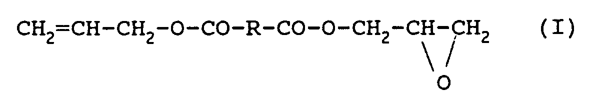 wherein R can be an aromatic ring diversely subsituted as phenyl or naphthyl or a linear or branched aliphatic chain formed by 4 to 20 carbon atoms, or norbornan or furan excluding 1-allyl-2-oxyran-2-ylmethyl-terephthalate.

2. Glycidyl esters according to claim 1 having the following chemical name:
1-allyl- 4-oxyran-2-ylmethylterephthalate, 1-allyl- 8-oxyran-2-ylmethyloctandioate, 1-allyl-3-oxyran-2-ylmethylterephthalate, 1-allyl-2-oxyran-2-ylmethylterephthalate, allylester, oxyran-2-ylmethylester of bicyclo [2.2.1]heptane-2,3-dicarboxylic acid, allylester,oxyran-2-ylmethylester of 2,5-furandicarboxylic acid.

3. Process for the preparation of glycidyl esters as defined in claims 1 and 2 which consists in the monoepoxidation of the corresponding diallylesters by reaction in phase transfer with hydrogen peroxide at 35% (m/v) and in the presence of catalysts based on tungsten.

4. Modified polyolefin which can be obtained by the radicalic grafting reaction in the molten state of a polyolefin having a density of at least 0.85 with 0.1-10% by weight with respect to the polyolefin of a glycidyl ester as defined in claims 1, also comprising 1-allyl-2-oxyran-2-ylmethyl-terephthalate, and 2 in the presence of 0.01-0.50% by weight of an initiator consisting of an organic peroxide.

5. Modified polyolefin according to claim 4 wherein the polyolefin is a copolymer of ethylene containing up to 50% of propylene, 1-butene, 4-methyl-1-pentene, 1-hexene or 1-octene and up to 5% of diolefins or triolefins such as ethylidenenorbornene, methylenenorbornene, 1,4-hexadiene vinylnorbornene and has a density of between 0.865 and 0.970.

6. Modified polyolefin according to claim 4 wherein the initiator is an organic peroxide represented by the formula (II) 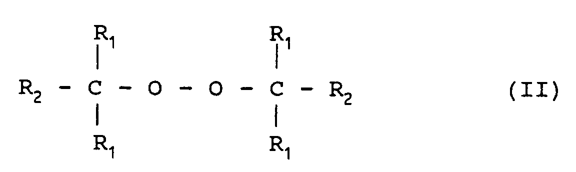 Wherein R₁ is a phenyl radical or alkyl radical containing from 1 to 10 carbon atoms, also unsaturated owing to the presence of double or triple C-C bonds, possibly substituted with alkyl or peroxyalkyl groups containing from 1 to 10 carbon atoms and R₂ is an alkyl radical containing from 1 to 10 carbon atoms.

7. Process for the preparation of a modified polyolefin as defined in claim 4 characterized in that 100 parts by weight of polyolefin are reacted, in the molten state, with 0.1-10 parts of glycidyl ester as defined in claims 1 and 2, for a time ranging from a few seconds to 20 minutes, at a temperature of between 160° and 200°C and in the presence of 0.01 - 0.5 parts of organic peroxide as defined in claim 6.
